# EUROPEAN PATENT APPLICATION

(11) **EP 4 767 961 A2**
(43) Date of publication of application: **01.07.2026**
(21) Application number: 25217160.8
(22) Date of filing: 20.11.2025
(51) Int. Cl.: A61B 18/12, A61B 18/14

(54) **ENCAPSULATED CATHETER WITH FRICTION-REDUCING SKIDS**

(30) Priority: 30.12.2024 US 202419005359
(71) Applicant: Biosense Webster (Israel) Ltd., 2066717 Yokneam (IL)
(72) Inventor: VAN NIEKERK, Pieter Emmelius, Irvine, 92618 (US); RODRIGUEZ SOTO, Juan, Irvine, 92618 (US); ABBAS, Mohammad, Irvine, 92618 (US); HIGHSMITH, Debby, Irvine, 92618 (US); EBRAHIMI, Babak, Irvine, 92618 (US)
(74) Representative: Carpmaels & Ransford LLP

(57) **Abstract**

The disclosed technology includes an end effector of a medical device. The end effector includes a framework, a flexible circuit including electrodes, an insulative material, and skids. The framework and flexible circuits are disposed on the insulative material. The insulative material includes a first surface disposed a first height from the framework in a height direction of the end effector and includes a first dynamic coefficient of friction. The skids are disposed on the insulative material, each skid comprising a second surface disposed a second height from the framework in the height direction and including a second dynamic coefficient of friction, lower than the first dynamic coefficient of friction. The first surface and the skids are disposed in the height direction such that the first height and each respective second height are one of equal or the respective second height is greater than the first height.

## Description

### FIELD

The present invention relates generally to minimally invasive medical devices, and in particular sensing catheters, and further relates to, but not exclusively, cardiac mapping catheters and the manufacture thereof.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Many times, electrode contact surfaces of these end effectors must be exposed through an encapsulating material via laser cutting or mechanical removal, both of which increase labor time and production costs.

### SUMMARY

To enable the time- and cost-efficient manufacture of end effectors that enhance the function of mapping and ablation catheters mentioned in the background section, the present disclosure relates to easily manufacturable end effectors, as well as fixtures and methods for manufacturing said end effectors having enhanced aspects of mapping and ablation catheter performance, including, but not limited to: mapping resolution, electrode contact with the target anatomy, delivery of the end effector to the target anatomy, biocompatibility, end effector stiffness, and atraumaticity.

There is provided, in accordance with the disclosed technology, an end effector of a medical device. The end effector includes a framework, a flexible circuit, an insulative material, and a plurality of skids. The flexible circuit comprises a plurality of electrodes. The framework and the flexible circuit are disposed on the insulative material. The insulative material comprises a first surface (i) that is disposed a first height from the framework in a height direction of the end effector and (ii) that comprises a first dynamic coefficient of friction. The plurality of skids are disposed on the insulative material, with each skid comprising a second surface (i) disposed a second height from the framework in the height direction and (ii) comprising a second dynamic coefficient of friction, lower than the first dynamic coefficient of friction. The first surface and the plurality of skids are disposed in the height direction such that the first height and each respective second height are one of equal or the respective second height is greater than the first height.

There is further provided, in accordance with the disclosed technology, an end effector of a medical device. The end effector comprises a framework, a flexible circuit, and an insulative material. The flexible circuit comprises a plurality of electrodes. The insulative material defines a first lumen, with the framework and the flexible circuit being disposed on the insulative material. The insulative material comprises a first sheet of insulative material comprising a first surface and a second surface and a second sheet of insulative material comprising a first surface and a second surface. The second surface of the first sheet and the second surface of the second sheet are joined such that the first lumen is defined at least partially by the second surface of the first sheet and the second surface of the second sheet.

There is further provided, in accordance with the disclosed technology, a method of forming an end effector of a medical device. The method comprises laminating a first sheet of insulative material and a second sheet of insulative material such that a framework and a flexible circuit are at least partially encapsulated therein. The method comprises forming a lumen that is defined at least partially by the first sheet and the second sheet.

There is further provided, in accordance with the disclosed technology, an end effector of a medical device. The end effector comprises a first framework, a second framework, a first flexible circuit, and an insulative material. The first flexible circuit is disposed between the first framework and the second framework and comprises a plurality of first electrodes. The first framework, the second framework, and the first flexible circuit are at least partially encapsulated by the insulative material.

### BRIEF DESCRIPTION OF THE DRAWINGS

FIG. 1 is a schematic pictorial illustration of a medical system including a medical device, in accordance with the disclosed technology;
FIG. 2 is a schematic pictorial illustration of a perspective view of an end effector, in accordance with the disclosed technology;
FIG. 3 is a schematic pictorial illustration of an exploded perspective view of the end effector of FIG. 2;
FIG. 4 is a schematic pictorial illustration of a cross-section of the end effector, taken along line 4-4 in FIG. 2, in accordance with the disclosed technology;
FIG. 5A is a schematic pictorial illustration of a cross-section of the end effector, taken along line 5A-5A in FIG. 2, in accordance with the disclosed technology;
FIG. 5B is a schematic pictorial illustration of a detail view of Detail A in FIG. 5A, in accordance with the disclosed technology;
FIG. 6 is a schematic pictorial illustration of a medical device assembly, in accordance with the disclosed technology;
FIG. 7 is a schematic pictorial illustration of a top view of the end effector collapsed within a sheath, in accordance with the disclosed technology;
FIG. 8 is a schematic pictorial illustration of a cross-section of an alternative configuration of an end effector, in accordance with the disclosed technology;
FIG. 9A is a schematic pictorial illustration of a cross-section of another alternative configuration of an end effector, in accordance with the disclosed technology;
FIG. 9B is a schematic pictorial illustration of a cross-section of an alternative configuration of the end effector of FIG. 9A, in accordance with the disclosed technology;
FIG. 10 a flowchart of a method of manufacturing an end effector for a medical device, in accordance with the disclosed technology; and
FIG. 11 is a schematic pictorial illustration of a cross-section of yet another alternative configuration of an end effector, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode medical device for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices for mapping and ablation of cardiac tissue to treat cardiac arrhythmias. Ablative energies are typically provided to cardiac tissue by a tip portion of a medical device (e.g., a medical probe or catheter) which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

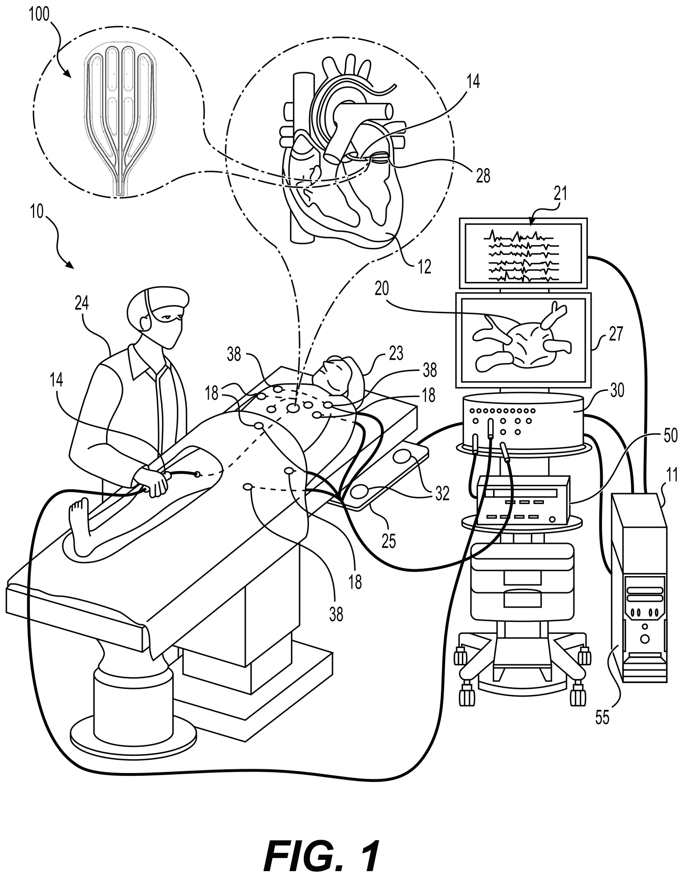

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a catheter shaft 90 with distal tip 28 of catheter 14 (i.e., multilayered end effector 100) into contact with the heart wall for sensing a target site in heart 12. For ablation, physician 24 would similarly bring a distal end of an ablation catheter to a target site for ablating.

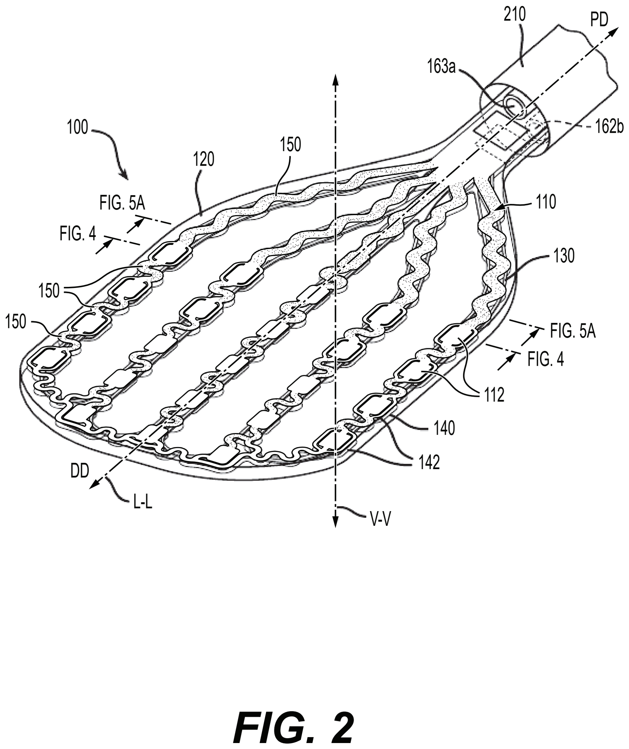

Catheter 14 is an exemplary catheter that includes one and preferably multiple electrodes 26 optionally distributed over end effector 100 coupled to a catheter shaft 90 and configured to sense the IEGM signals as described in more detail below. Catheter 14 may additionally include a position sensor (as shown in FIG. 2B) embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26 at a distal tip 28 of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

FIG. 2 provides an end effector 100 in accordance with an example of the present disclosure in order to achieve the ease of manufacturing, reduced cost, and enhanced end effector properties, such as desired collapsibility, maneuverability, robustness, stiffness, mapping resolution, electrode contact with target anatomy, and conformity of the end effector 100 disclosed herein to flat, curved, irregular and/or nonplanar tissue surfaces found in the target anatomy. The end effector 100 extends along a longitudinal axis L-L and can include a flexible circuit 110 including a plurality of electrodes 112, each electrode of the plurality of electrodes 112 including a contact surface 112c. As used herein, the term "flexible circuit" includes thin-film circuit, flexible printed circuit board, thin film deposition via lithography and etching processes on substrate such as polyimide, copper, LCP (e.g., Panasonic FELIOS^{™} SERIES of Flexible Circuit Boards), nitinol substrate or directly onto the polymeric substrate such as Panasonic BEYOLEX^{™} PRINTED ELECTRONICS SUBSTRATE Series as shown and described in the attached technical references incorporated herein by the Appendix. In some examples, the flexible circuits described herein can be made primarily of polyimide. In other examples, it can be made of any of biocompatible polyimides, glass-reinforced epoxy laminate materials, copper, or graphene, alone or in combination. The first 110 and second 140 flexible circuits can include conductive traces directly formed onto the polymeric substrate with the electrode comprising a substantially much thicker version of the traces such that the typical polyimide substrate is no longer required. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuit 110 can be disposed on an insulative material 120 (also known as an insulative mass). The insulative material 120 can be contiguous to the contact surfaces 112c so that only the contact surfaces 112c of at least a portion of the plurality of electrodes 112 are exposed to the ambient environment. As used herein, the term "contact surface" includes the portion of an electrode having a generally flat surface and the edge or edges which immediately surround said flat surface. Electrodes 112 may have a slightly rounded, radiused, or chamfered edge that comes into contact with tissue, along with the generally flat surface, when the end effector 100 is placed against tissue. As used herein, "ambient environment" refers to the external environment such as the organ in which the end effector 100 is deployed or in the operating theater prior to being deployed in the biological organ.

It is noted that not all of the electrodes on the end effector 100 described herein need be exposed through the insulative material 120 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 163a on one side and 162b on the other side in fluid communication with an irrigation line not shown disposed in a catheter shaft 90. Instead of an irrigation line separate from the catheter shaft 90, a lumen can be formed via extrusion of the catheter shaft 90 to provide for a lumen channel. It is noted that port 163a or 162b can be configured to have a sufficient flow diverter characteristic for irrigation fluid to cover the mapping electrodes during irrigation flow so as to prevent or reduce thrombus formations.

The end effector 100 can further include a framework 130 contiguous to the insulative material 120 or at least partially encapsulated in the insulative material 120. In examples in which the end effector 100 includes framework 130, the framework 130 can be spaced from the flexible circuit 110 by some of the insulative material 120 coming therebetween.

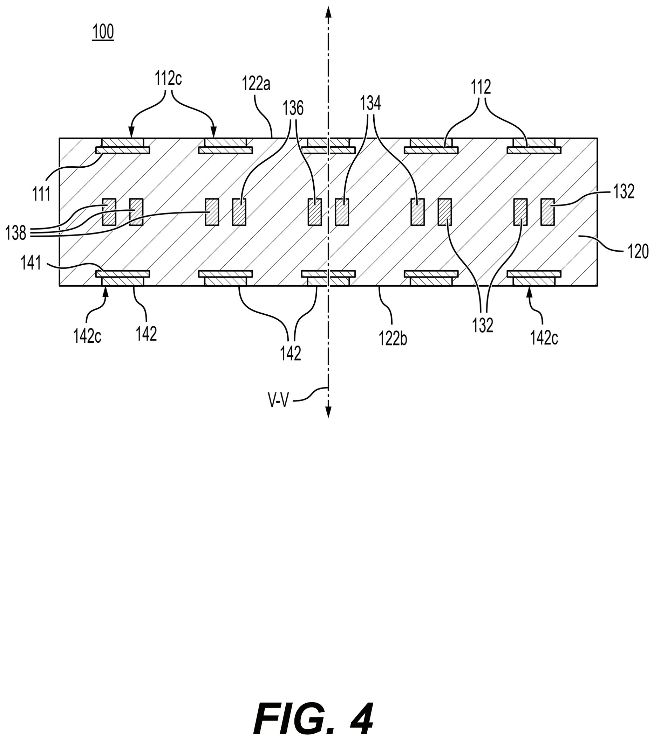

Stated otherwise, an aspect of the present disclosure provides an end effector 100 having a planar framework 130 bisecting two flat, heat formed portions 120a, 120b of a flexible insulating mass 120, with at least one flexible circuit 110 disposed on one side of the framework 130 and with contact surfaces 112c of electrodes 112 extending up to (and flush with, as seen in FIG. 4), slightly past, or slightly recessed relative to the outer face of the flexible insulating mass 120.

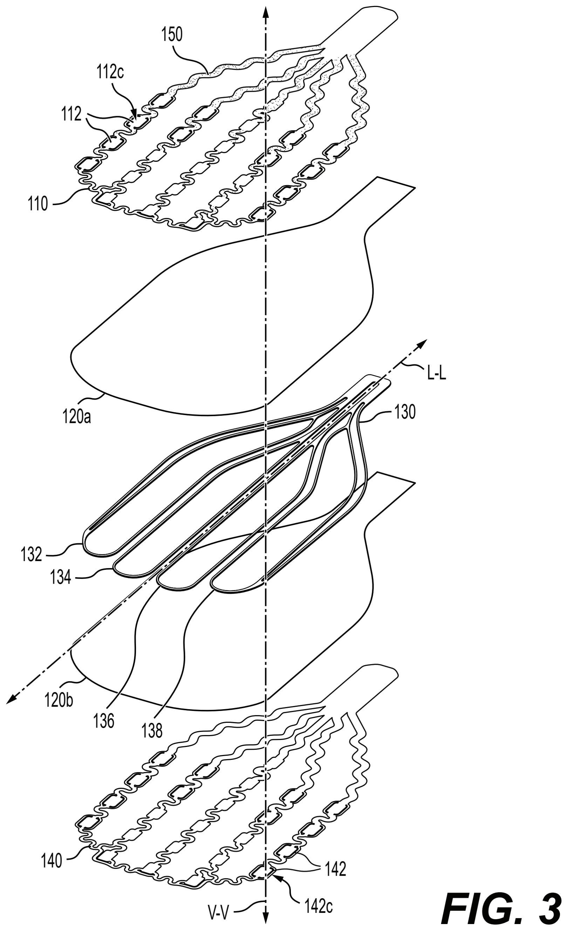

FIG. 3 shows an exploded view of the end effector 100, with the components thereof exploded vertically along vertical axis V-V (also referred to as the height direction) of the end effector 100. The flexible circuit 110 can be a first flexible circuit 110 and the plurality of electrodes 112 can be a plurality of first electrodes 112, with each first electrode 112 including a first contact surface 112c. The end effector 100 can further include a second flexible circuit 140 having a plurality of second electrodes 142. The second flexible circuit 140 can be spaced apart from the first flexible circuit 110 and each electrode of the second plurality of electrodes 142 can have a second contact surface 142c. The contact surfaces 112c, 142c, in use, are configured to either contact or be disposed proximal to tissue. Moreover, each flexible circuit 110, 140 includes respective tines 111, 141 extending along the longitudinal axis L-L, with the tines 111, 141 supporting the respective electrodes 112, 142 (see, for example, FIG. 4).

In examples having first 110 and second 140 flexible circuits, the insulative material 120 can be disposed between the first flexible circuit 110 and the second flexible circuit 140, and the insulative material 120 can be contiguous to the second contact surfaces 142c so that only the contact surface 142c of each second electrode 142 is exposed to the ambient environment, similar to how first electrodes 112 are disposed in and exposed through the insulative material 120.

Electrodes 112, 142 can sense tissue signals or transmit energy AC or DC from an energy generator to the tissues. In some examples, there are about 92 electrodes. In some examples, there are about 48 electrodes. In some examples, there are about 64 electrodes. In some examples, there are about 72 electrodes. In some examples, there are about 98 electrodes. However, those skilled in the art will apricated that various numbers of electrodes 112, 142 can be employed without departing from the spirit and scope of the present disclosure.

In examples herein, the end effector 100 includes a framework 130 disposed between the first flexible circuit 110 and the second flexible circuit 140. Framework 130 can be a component of the end effector 100 that is separate and distinct from the first flexible circuit 110 and disposed proximate the first flexible circuit 110. In this case, the insulative material 120 can be further disposed between the framework 130 and the second flexible circuit 140. The framework 130 can be formed from a planar or cylindrical stock of material using any suitable method. For example, the framework 130 can be formed by cutting, laser cutting, stamping, etc.

The framework includes a plurality of spines 132, 134, 136, 138 extending along the longitudinal axis L-L. The tines 111, 141 of the flexible circuits 110, 140 can be approximately aligned with one or more of the spines 132, 134, 136, 138 such that the spines 132, 134, 136, 138 provide support thereto.

Insulative material 120 can include a first sheet of insulative material 120a and a second sheet of insulative material 120b fused together proximate the framework 130 into a single, contiguous, generally planar insulative material 120. This insulative material 120 also serves to enhance the atraumaticity of the end effector 100 and to protect the subject from sharp edges.

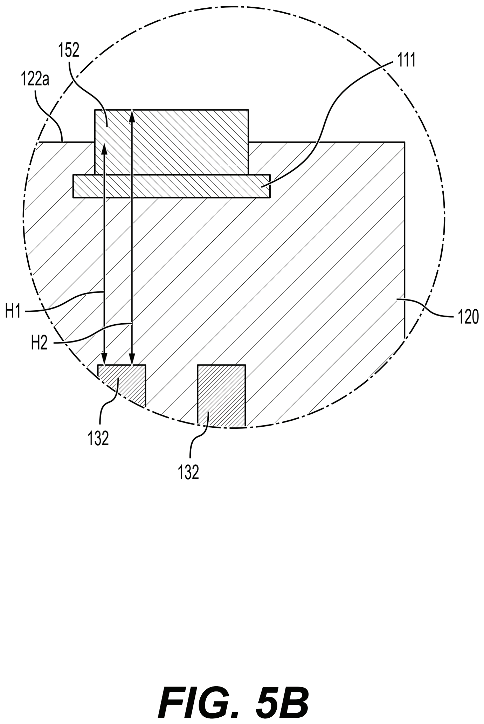

FIGs. 4-5B depict various cross-sectional and detail views of the end effector 100. As seen in FIGs. 4-5A, the insulative material 120 includes opposing outer surfaces 122a (referred to herein as a first insulative surface 122a), 122b (referred to herein as a second insulative surface 122b). In some examples, the first and second outer surfaces 122a, 122b extend in approximately parallel planes. The first surface 122a and the second surface 122b are both disposed a first height H1 (FIG. 5B) from the framework 130 and longitudinal axis L-L along the vertical axis V-V.

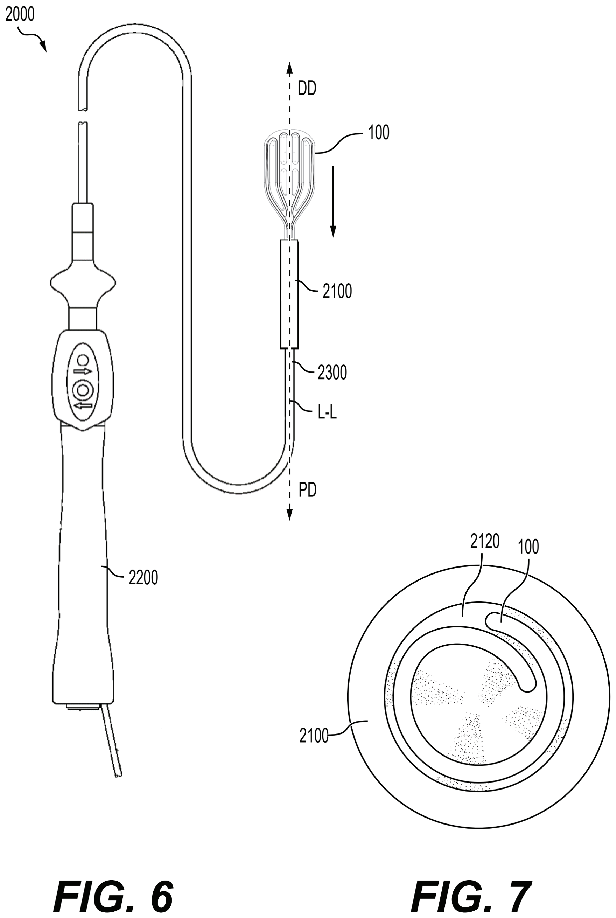

The insulative material 120 can include polymer. The insulative material 120 can be heat formed around at least a portion of the first flexible circuit 110, the second flexible circuit 140, and the framework 130. The polymer can include thermoplastic polyurethane (TPU) or other heat formed or shaped material which lends itself to said heat forming, such as, but not limited to, silicone or siloxane. In the examples described herein, the insulative material 120 comprises a high friction material. As used herein, the term "high friction material" is used to differentiate from other materials that have a lower dynamic coefficient of friction between the other materials and a predetermined material/object (e.g., a sheath or vascular structure) relative to the dynamic coefficient of friction between the high friction material and the predetermined material/object. The term "low friction material" (discussed in greater detail below relative to skids 150) is used herein to describe those other materials that have lower coefficients of friction compared to the high friction material when measured in contact with the same predetermined material/object. In some examples, the outer surfaces 122a, 122b of the insulative material 120 and a sheath 210 (FIG. 6, discussed in greater detail below, which is an example of a predetermined object, and is also referred to in the art as an introducer tool) have a dynamic coefficient of friction between approximately .02 and .6, or a median value of approximately .31. By way of example, the sheath can be made from any number of low friction materials, such as but not limited to, high-density polyethylene, low-density polyethylene, a polyether block amide (known under the tradename of Pebax^{™}) with a lubricious layer (e.g., using EverGlide^{™}), polytetrafluorethylene, and the like.

Furthermore, while the insulative material 120 is shown to be flat in these figures, insulative material 120 can be shaped, scalloped, ribbed, ridged, concaved, convexed, or otherwise configured such that the overall profile of insulative material 120 yields physical and/or mechanical properties, such as rigidity and flexion along multiple axes, required by the end effector 100, mentioned above.

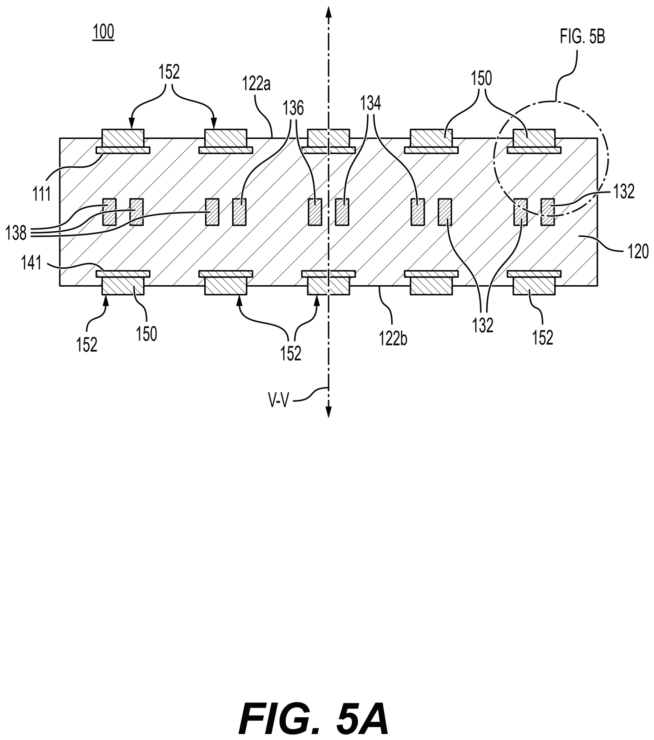

With continued to reference to FIGs. 2-5B, the end effector 100 further includes skids 150. The framework 130, the flexible circuits 110, 140, and the skids 150 being stacked along the height direction/vertical axis V-V. The framework 130 and the flexible circuits 110, 140 are at least partially encapsulated by the insulative material 120, with the skids 150 either being disposed on an outer surface the insulative material 120 or partially encapsulated by the insulative material 120. For example, and as seen in FIGs. 5A-5B, an inner surface of each skid 150 can be disposed directly on one of the flexible circuits 110, 140 and extending away therefrom along the height direction.

Further to the above, the skids 150 can be provided along one or more of the tines 111, 141 of the respective flexible circuits 110, 140, as shown in FIG. 5A. In the example depicted, each tine 111, 141 is provided with a corresponding skid 150, with each skid 150 being discontinuous such that the electrodes 112, 142 are not covered by the skids 150. However, those skill in the art will appreciate that the skids 150 can take any appropriate configuration without departing from the spirit and scope of the present disclosure. For example, the skids 150 can be provided on distinct tines 111, 141 that do not include electrodes (i.e., those tines 111, 141 are provided specifically to engage with and support skids 150). Alternatively, the skids 150 can be encapsulated/connected (e.g., overlayed or embedded) with the insulative material 120 in a manner that does not employ a direct connection with the flexible circuits 110, 140.

The skids 150 are configured to create reduced friction contact surfaces of the end effector 100 during insertion into a patient's body, retraction into a sheath 210 (FIG. 7), and manipulation and storage of the catheter 14. By doing so, the amount of force required to control movement of the end effector 100 can be reduced.

Each skid 150 has an outer surface 152 that is disposed a second height H2 (FIG. 5B) from the framework 130 and longitudinal axis L-L. The second height H2 is either equal to greater than the first height H1, resulting in the outer surface 152 of each skid 150 either being flush with or protruding from the outer surfaces 122a, 122b of the insulative material 120. For example, the skids 150 can be directly disposed on one of the respective flex circuits 110, 140 and extend, in the height direction V-V, through one of the outer surfaces 122a, 122b of the insulative material 120, and terminate at the skid outer surface 152.

The number of skids 150 protruding from or being flush with the outer surfaces 122a, 122b of the insulative material can be varied, in accordance with the disclosed technology, depending on the design requirements of the end effector. In instances where at least some of the skids 150 protrude from the outer surfaces (i.e., H2 is greater than H1), the amount of protrusion can be in the range of approximately no protrusion (i.e., the difference between H2 and H1 is approximately zero) to approximately 5-20 microns, as the intent of the skids 150 is to change the surface interaction between the end effector 100 and the predetermined object/material, not increase material. In some examples, all of the skids 150 are flush with the outer surfaces 122a, 122b of the insulative material 120 (i.e., H2 equals H1). In some examples, all of the skids 150 protrude from the outer surfaces 122a, 122b of the insulative material 120 (i.e., H2 is greater than H1). In some examples, there is a mix of skids 150 that are flush with and protrude from the outer surfaces 122a, 122b of the insulative material 120.

Moreover, each skid 150 is formed from a low friction material such that contact between the outer surface 152 of each skid and the aforementioned predetermined object/material (e.g., sheath 210) results in a lower dynamic coefficient of friction than the dynamic coefficient of friction between the insulative material 120 and the predetermined object/material. In some examples, the outer surfaces 152 of the skids 150 and a sheath 210 (FIG. 6, discussed in greater detail below, which is an example of a predetermined object) have a dynamic coefficient of friction on the lower end of the aforementioned range described with respect to the insulative material 120 and the predetermined material/object. For example, the dynamic coefficient between the skids 150 and the predetermined material/object can be between approximately .02 and .3, which is reduced relative to the range of coefficient of frictions of the insulative material 120. Put another way, a median value of this range (e.g., approximately .16) is less than a median value of the range of values of the dynamic coefficient of the insulative material 120 (e.g., approximately .31, as discussed above). In general, the skids 150 are designed such that their dynamic coefficient of frictions (or their range of dynamic coefficient of frictions) are less than that of the insulative material 120 relative to their contact with the same material/object.

In some examples, the low friction material of each skid 150 can include polyimide, liquid crystal polymer (LCP), ethylene tetrafluoroethylene (ETFE), polyether ether ketone (PEEK), and combinations thereof. However, it will be appreciated by those skilled in the art that the low friction material can include any appropriate biocompatible material with low friction properties (other than, or in conjunction with the materials listed in the preceding sentence) without departing from the spirit and scope of the present disclosure.

Because of the skids' either flush or protruding configuration from the insulative material 120, in conjunction with its' lower friction material relative to the insulative material 120, overall frictional forces on the end effector can be reduced.

An exemplary use case is shown in FIGs. 6-7, which depict a medical probe assembly 2000. The medical probe assembly 2000 includes a tubular member 2300 extending along a longitudinal axis 60 and is configured to deliver any of the end effectors 100 previously discussed to, out of, and/or back into a sheath 2100. As seen particularly in FIG. 7, when the end effector 100 moves into a lumen 2120 of the sheath 2100, the skids 150 engage the wall of the lumen 2120, thereby providing a low-friction interface therewith to facilitate the collapse of the end effector 100. The skids 150 also aid in allowing the lateral ends of the end effector 100 to Moreover, it is noted that the physician 24 can manipulate the medical probe assembly 2000 with handle 2200. Appropriate examples for catheter assembly 2000 and its subcomponents such as handle 2200, sheath 2100, tubular member 2300, and others not mentioned herein are described in US Patent publication No. 2021/0369339, which is incorporated herein by reference.

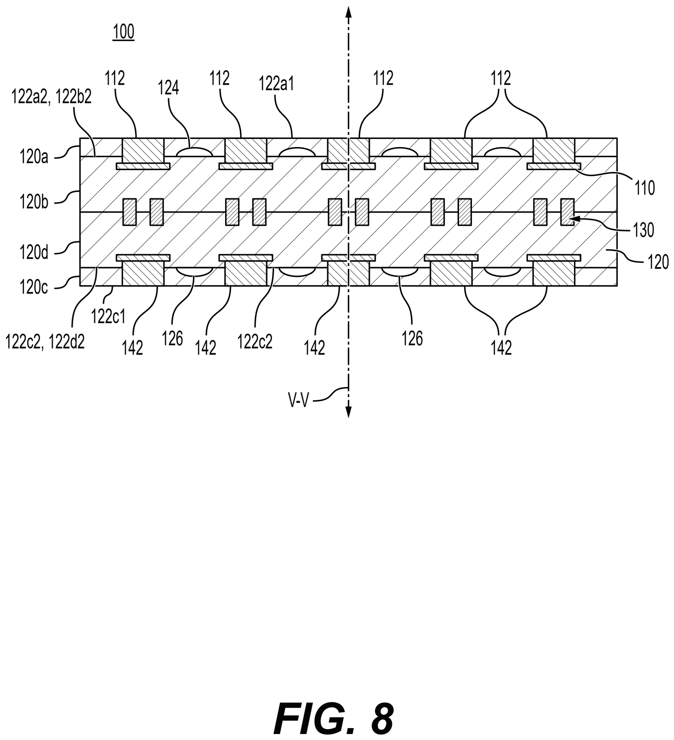

FIGs. 8-9B depicts other aspects of the present disclosure. Irrigation is typically required in medical catheters for various reasons, such as, but not limited to, for cooling and/or temperature control (e.g., during ablation) and for the prevention of clot formation. The designs illustrated in these figures can be employed with similar (or the same) concepts as previously described (e.g., with skids 150, flexible circuits 110, 140, and electrodes 112, 142). Therefore, details specific to the configurations shown in FIGs. 8-9B are focused on in this section.

FIG. 8 depicts a cross-sectional view of a multilayered end effector 100 that includes a first flexible circuit 110, first electrodes 112, insulative material 120, a framework 130, a second flexible circuit 140, and second electrodes 142. The insulative material 120 similarly encapsulates and/or spaces the components of the end effector 100, as discussed in previous examples. In this configuration, the insulative material 120 is formed from four insulative sheets: a first sheet 120a, a second sheet 120b, a third sheet 120c, and a fourth sheet 120d that are heat formed together to form one contiguous mass. The framework 130 is disposed on and sandwiched by the second flexible circuit 120b and the fourth flexible circuit 120d. The flexible circuits 110, 140 are shown as being disposed on the second flexible circuit 120b and the fourth flexible circuit 120d, respectively, but, as will be appreciated by those skilled in the art, the flexible circuits 110, 140 can be disposed on any appropriate layer/sheet of the insulative material 120 without departing from the spirit and scope of the present disclosure.

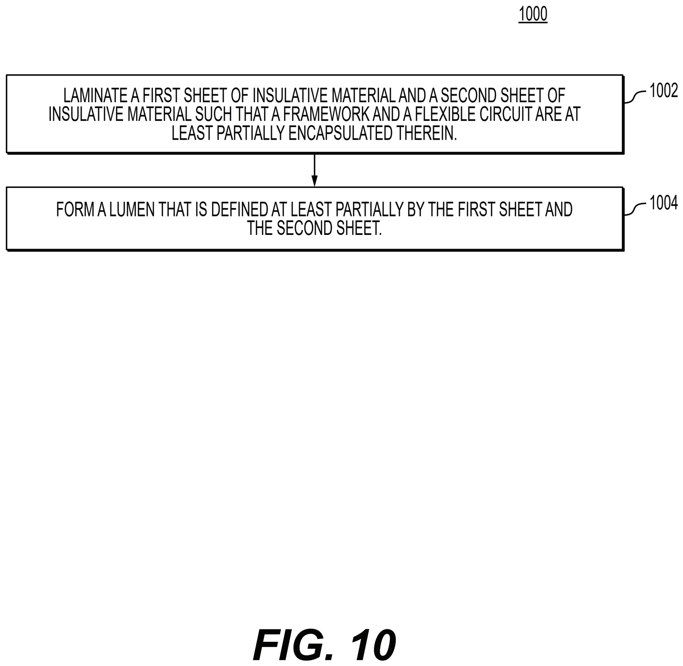

The insulative material 120 defines one or more first lumens 124 that are formed between the adjacent first and second sheets 120a, 120b. Depending on the irrigation requirements of the system, one or more second lumens 126 can additionally be formed between the adjacent third and fourth sheets 120c, 120d. These lumens 124, 126 can extend from a proximal end of the end effector 100 to the distal end thereof and/or include channels formed through the outermost sheets 120a, 120c at predetermined locations along the longitudinal axis L-L. Methods of forming these lumens 124, 126 are discussed in greater detail with respect to FIG. 10.

The first sheet 120a has a first surface 122a1 and an opposite second surface 122a2. The second sheet 120b has a first surface 122b1 and an opposite second surface 122b2. The third sheet 120c has a first surface 122c1 and an opposite second surface 122c2. The fourth sheet 120d has a first surface 122d1 and an opposite second surface 122d2. The second surface 122a2 of the first sheet 120a and the second surface 122b2 of the second sheet 120b are joined such that the first lumens 124 are defined at least partially by the second surface 122a2 of the first sheet 120a and the second surface 122b2 of the second sheet 120b. Similarly, the second lumens 126 are formed at least partially by the joined second surfaces 122c2, 122d2 of the third and fourth sheets 120c, 120d.

In the present example, the lumens 124, 126 are formed as recesses in one of the second surfaces 122a2, 122b2, 122c2, 122d2. They can be formed by any suitable process such as, for example, mechanical or laser formation processes. Alternatively, the lumens 124, 126 can be formed by selectively leaving one or more regions of the insulative material 120 unlaminated (i.e., not heat formed).

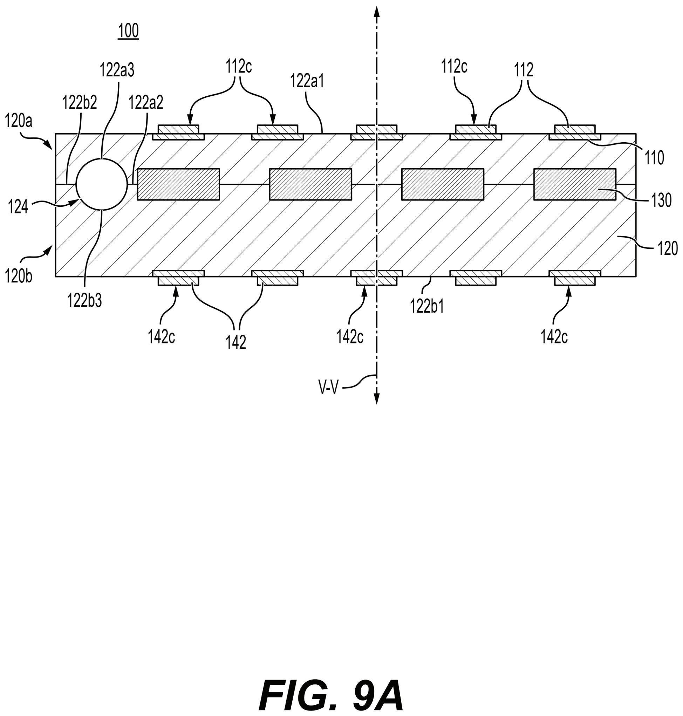

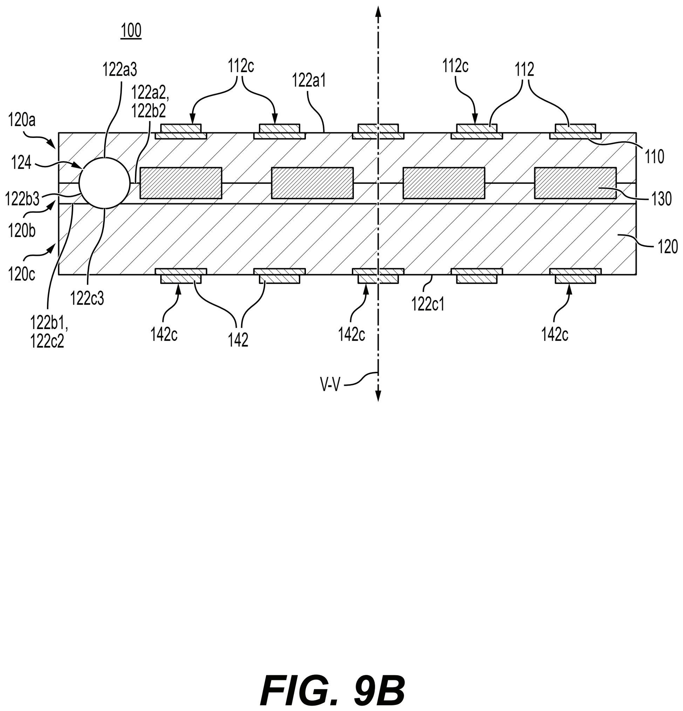

FIG. 9B depicts as similar concept as that of FIG. 9A, but with a different configuration of insulative material 120 and lumen 124 that demonstrates how an alternative approach that employs the concepts discussed herein. In this example, three sheets 120a, 120b, 120c are heat formed together to form the insulative material/mass 120. A single lumen 124 is formed by recesses 122a3, 122b3, 122c3 formed in the second surfaces 122a2, 122b2, 122c2 of the respective sheets 120a, 120b, 120c such that the three sheets 120a, 120b, 120c collectively define the lumen 124. In this example, the lumen 124 is positioned laterally relative to the framework 130 and flexible circuits 110, 140.

FIG. 10 depicts a flow chart of one or more methods 1000 of forming an end effector. The method(s) 1000 generally include the following steps. A first sheet of insulative material and a second sheet of insulative material are laminated 1002 such that a framework and a flexible circuit are at least partially encapsulated therein. A lumen is formed 1004 that is defined at least partially by the first sheet and the second sheet.

In some examples, the lumen is formed 1004 by selectively laminating the first sheet and the second sheet such that a non-laminated section of the first sheet and the second sheet defines the lumen. For example, the end effector 100 of FIG. 8 can have the lumens 124 created by selectively laminating the sheets 120a, 120b (e.g., via laser welding) at their abutting surfaces 122a2, 122b2, with non-laminated sections forming voids that function as irrigation lumens 124.

In some examples, the lumen is formed 1004 by forming a recess in one of the sheets (e.g., the first sheet). This can be done by any appropriate process, such as laser cutting. See, e.g., the recesses formed in FIG. 8.

In some examples, the lumen is formed 1004, in part, by positioning a second material with a higher melting point than the insulative material between the first sheet and the second sheet. By way of example, the second material can include, but is not limited to polytetrafluoroethylene (PTFE), ethylene tetrafluoroethylene (ETFE), polyimide, and the like. FIGs. 9A and 9B depicts an examples of this process, where a hollow cylindrical and/or tubular second material is used to form a cylindrical irrigation lumen 124 at the boundary where the first and second sheets 120a, 120b are joined. Besides tubular shapes, two or more flat/rectangular strips can positioned with a gap therebetween to form a rectangular shaped lumen.

In some examples where a second material is employed (e.g., polyether ether ketone (PEEK), polished stainless steel or the like), the two sheets can be laminated and the second material removed thereafter, leaving a void as the lumen. In other words, the second material is only temporarily a part of the end effector 100 structure as a portion of the manufacturing process.

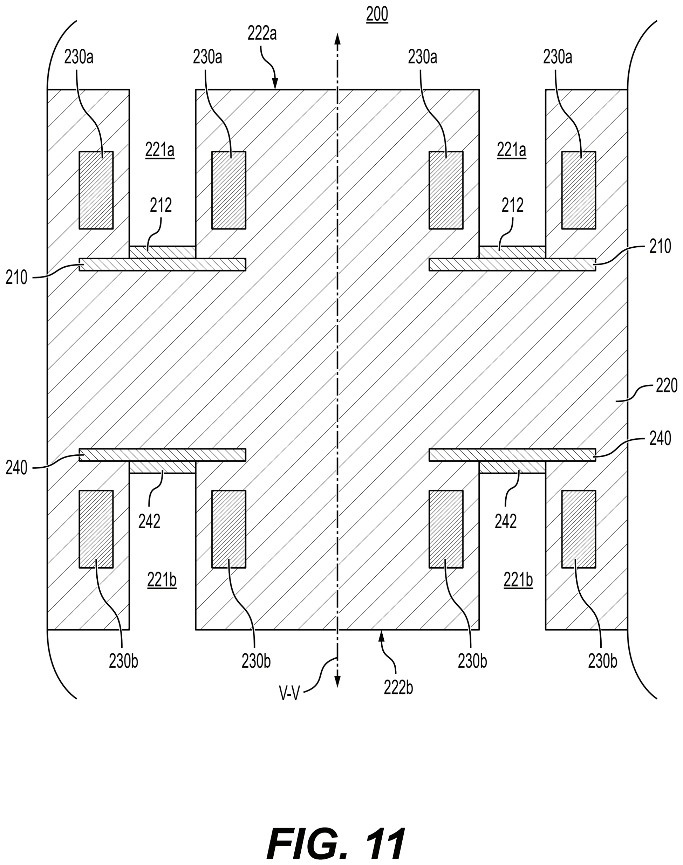

FIG. 11 depicts yet another aspect of the present disclosure. As mentioned above, the end effector 100 experiences many forces over the course of its use. The end effector 200 depicted in FIG. 11 employs a reverse configuration of the framework 230a, 230b relative to the flexible circuits 210, 240. The designs illustrated in these figures can be employed with similar (or the same) concepts as previously described (e.g., with skids 150, lumens 124, a planar framework in form with tines as seen in FIG. 3, etc.). Therefore, details specific to the configuration shown in FIG. 11 are focused on in this section.

The end effector includes a first framework 230a, a second framework 230b, a first flexible circuit 210, a second flexible circuit 240, and an insulative material 220. The frameworks 230a, 230b can be formed from a super elastic material, such as nitinol. The first flexible circuit 210 is disposed between the first framework 230a and the second framework 230b along the vertical axis V-V and includes a plurality of first electrodes 212. The second flexible circuit 240 is also disposed between the first framework 230a and the second framework 230b along the aforementioned vertical axis and includes a plurality of second electrodes 242. The insulative material 220 at least partially encapsulates the first framework 230a, second framework 230b, first flexible circuit 210, and second flexible circuit 240. With the framework in this example being configured as two framework sections 230a, 230b that are disposed further away from the center of the insulative material 220 (and end effector 200) along the vertical axis V-V than the flexible circuits 210, 240, the framework 230a, 230b can protect against the high bending forces that are experienced by the end effector 200 in use.

Additionally, a plurality of first gaps 221a are formed in the insulative material 220. Each first gap 221a extends from an outer surface 222a of the insulative material 220, through tines of the first framework 230a, and aligns with and exposes one of the first electrodes 212. Similarly, on an opposing side of the end effector 200, a plurality of second gaps 221b are formed in the insulative material 220. Each second gap 221b extends from another outer surface 222b of the insulative material 220 (opposite the outer surface from which the first gaps 221a extend), through tines of the second framework 230b, and aligns with and exposes one of the second electrodes 242. These gaps 221a, 221b enable exposure of the electrodes 212, 242 to the outside environment for cardiac mapping and/or ablation.

The disclosed technology described herein can be further understood according to the following clauses:
Clause 1. An end effector of a medical device comprising: a framework; a flexible circuit comprising a plurality of electrodes; an insulative material, the framework and the flexible circuit being disposed on the insulative material, and the insulative material comprising a first surface (i) disposed a first height from the framework in a height direction of the end effector and (ii) comprising a first dynamic coefficient of friction; and a plurality of skids disposed on the insulative material, each skid comprising a second surface (i) disposed a second height from the framework in the height direction and (ii) comprising a second dynamic coefficient of friction, lower than the first dynamic coefficient of friction, the first surface and the plurality of skids being disposed in the height direction such that the first height and each respective second height are one of equal or the respective second height is greater than the first height.
Clause 2. The end effector of clause 1, at least some of the second heights being greater than the first height.
Clause 3. The end effector of clause 2, all of the second heights being greater than the first height.
Clause 4. The end effector of any one of clauses 2-3, a difference between the second height and the first height being in the range of five to twenty microns.
Clause 5. The end effector of clause 1, at least some of the second heights being equal to the first height.
Clause 6. The end effector of clause 5, all of the second heights being equal to the first height.
Clause 7. The end effector of any one of clauses 1-6, the dynamic coefficient of friction of the first surface comprising a value up to approximately .6.
Clause 8. The end effector of clause 7, the dynamic coefficient of friction of each respective second surface being between approximately .02 and .3, and less than the value of the dynamic coefficient of friction of the first surface.
Clause 9. The end effector of any one of clauses 1-8, the insulative material comprising at least one of thermoplastic polyurethane, silicone, or siloxane.
Clause 10. The end effector of any one of clauses 1-9, the framework, the flexible circuit, and the skids being stacked along the height direction, with the framework and the flexible circuit being at least partially encapsulated by the insulative material.
Clause 11. The end effector of any one of clauses 1-10, the skids being at least partially encapsulated by the insulative material.
Clause 12. The end effector of any one of clauses 1-11, each skid being disposed on the flex circuit and extending, in the height direction, from the flex circuit, through the first surface, and terminate at the second surface.
Clause 13. The end effector of any one of clauses 1-12, the first dynamic coefficient of friction and the second dynamic coefficient of friction each comprising a value or a range of values respectively configured to be determined by dynamic contact between the insulative material and a predetermined object and dynamic contact between each skid and the predetermined object.
Clause 14. The end effector of clause 13, the predetermined object comprising a sheath.
Clause 15. An end effector of a medical device, the end effector comprising: a framework; a flexible circuit comprising a plurality of electrodes; an insulative material defining a first lumen, the framework and the flexible circuit being disposed on the insulative material, and the insulative material comprising: a first sheet of insulative material comprising a first surface and a second surface; and a second sheet of insulative material comprising a first surface and a second surface, the second surface of the first sheet and the second surface of the second sheet being joined such that the first lumen is defined at least partially by the second surface of the first sheet and the second surface of the second sheet.
Clause 16. The end effector of clause 15, the second surface of the first sheet comprising a recess, the recess defining a portion of the first lumen.
Clause 17. The end effector of any one of clauses 15-16, the second surface of the second sheet comprising a recess, the recess defining a portion of the first lumen.
Clause 18. The end effector of any one of clauses 15-17, the insulative material further defining a second lumen and comprising: a third sheet of insulative material comprising a first surface and a second surface; and a fourth sheet of insulative material comprising a first surface and a second surface, the second surface of the third sheet and the second surface of the fourth sheet being joined such that the second lumen is defined by the second surface of the first sheet and the second surface of the second sheet.
Clause 19. The end effector of any one of clauses 15-17, the insulative material further comprising: a third sheet of insulative material comprising a first surface and a second surface, the first lumen being defined at least partially by the second surface of the third sheet.
Clause 20. The end effector of any one of clauses 15-19, further comprising: a plurality of skids disposed on the insulative material, each skid comprising a second surface comprising a second dynamic coefficient of friction, lower than a first dynamic coefficient of friction of the first surface of the first sheet.
Clause 21. The end effector of any one of clauses 15-20, the first lumen being configured to deliver irrigation fluid therethrough.
Clause 22. A method of forming an end effector of a medical device, the method comprising: laminating a first sheet of insulative material and a second sheet of insulative material such that a framework and a flexible circuit are at least partially encapsulated therein; and forming a lumen that is defined at least partially by the first sheet and the second sheet.
Clause 23. The method of clause 22, the forming a lumen comprising: selectively laminating the first sheet and the second sheet such that a non-laminated section of the first sheet and the second sheet defines the lumen.
Clause 24. The method of clause 22, the forming a lumen comprising: forming a recess in the first sheet that at least partially defines the lumen.
Clause 25. The method of clause 24, the forming a recess comprising: laser cutting the first sheet to form the recess.
Clause 26. The method of clause 22, the forming a lumen comprising: positioning a second material with a higher melting point than the insulative material between the first sheet and the second sheet.
Clause 27. The method of clause 26, the second material comprising a tubular shape.
Clause 28. The method of clause 26, the second material comprising a pair of flat strips that define a gap therebetween.
Clause 29. The method of clause 26, further comprising, after the laminating a first sheet of insulative material and a second sheet of insulative material: removing the second material to define the lumen.
Clause 30. An end effector of a medical device, the end effector comprising: a first framework; a second framework; a first flexible circuit disposed between the first framework and the second framework and comprising a plurality of first electrodes; and an insulative material, the first framework, the second framework, and the first flexible circuit being at least partially encapsulated by the insulative material.
Clause 31. The end effector of clause 30, the first framework and the second framework comprising a super elastic material.
Clause 32. The end effector of any one of clauses 30-31, further comprising a plurality of first gaps formed in the insulative material and extending through the first framework, each first gap being aligned with a respective first electrode.
Clause 33. The end effector of any one of clauses 30-32, further comprising a second flexible circuit disposed between the first framework and the second framework and comprising a plurality of second electrodes.
Clause 34. The end effector of clause 33, further comprising a plurality of second gaps formed in the insulative material and extending through the second framework, each second gap being aligned with a respective second electrode.
Clause 35. The end effector of any one of clauses 30-34, further comprising: a plurality of skids disposed on the insulative material, each skid comprising a second surface comprising a second dynamic coefficient of friction, lower than a first dynamic coefficient of friction of a first surface of the insulative material.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. An end effector of a medical device comprising:
a framework;
a flexible circuit comprising a plurality of electrodes;
an insulative material, the framework and the flexible circuit being disposed on the insulative material, and the insulative material comprising a first surface (i) disposed a first height from the framework in a height direction of the end effector and (ii) comprising a first dynamic coefficient of friction; and
a plurality of skids disposed on the insulative material, each skid comprising a second surface (i) disposed a second height from the framework in the height direction and (ii) comprising a second dynamic coefficient of friction, lower than the first dynamic coefficient of friction,
the first surface and the plurality of skids being disposed in the height direction such that the first height and each respective second height are one of equal or the respective second height is greater than the first height.

2. The end effector of claim 1, at least some of the second heights being greater than the first height.

3. The end effector of claim 2, all of the second heights being greater than the first height, and/or a difference between the second height and the first height being in the range of five to twenty microns.

4. The end effector of claim 1, at least some of the second heights being equal to the first height, optionally all of the second heights being equal to the first height.

5. The end effector of claim 1, the dynamic coefficient of friction of the first surface comprising a value up to approximately .6.

6. The end effector of claim 5, the dynamic coefficient of friction of each respective second surface being between approximately .02 and .3, and less than the value of the dynamic coefficient of friction of the first surface.

7. The end effector of any preceding claim, the framework, the flexible circuit, and the skids being stacked along the height direction, with the framework and the flexible circuit being at least partially encapsulated by the insulative material.

8. The end effector of any preceding claim, the skids being at least partially encapsulated by the insulative material.

9. The end effector of any preceding claim, each skid being disposed on the flex circuit and extending, in the height direction, from the flex circuit, through the first surface, and terminate at the second surface.

10. The end effector of any preceding claim, the first dynamic coefficient of friction and the second dynamic coefficient of friction each comprising a value or a range of values respectively configured to be determined by dynamic contact between the insulative material and a predetermined object and dynamic contact between each skid and the predetermined object, optionally the predetermined object comprising a sheath.

11. An end effector of a medical device, the end effector comprising:
a framework;
a flexible circuit comprising a plurality of electrodes;
an insulative material defining a first lumen, the framework and the flexible circuit being disposed on the insulative material, and the insulative material comprising:
a first sheet of insulative material comprising a first surface and a second surface; and
a second sheet of insulative material comprising a first surface and a second surface,
the second surface of the first sheet and the second surface of the second sheet being joined such that the first lumen is defined at least partially by the second surface of the first sheet and the second surface of the second sheet.

12. The end effector of claim 11, the second surface of the first sheet comprising a recess, the recess defining a portion of the first lumen, and/or the second surface of the second sheet comprising a recess, the recess defining a portion of the first lumen.

13. An end effector of a medical device, the end effector comprising:
a first framework;
a second framework;
a first flexible circuit disposed between the first framework and the second framework and comprising a plurality of first electrodes; and
an insulative material,
the first framework, the second framework, and the first flexible circuit being at least partially encapsulated by the insulative material.

14. The end effector of claim 13, further comprising a plurality of first gaps formed in the insulative material and extending through the first framework, each first gap being aligned with a respective first electrode.

15. The end effector of any of claims 11 to 14, further comprising:
a plurality of skids disposed on the insulative material, each skid comprising a second surface comprising a second dynamic coefficient of friction, lower than a first dynamic coefficient of friction of a first surface of the insulative material.
